# EUROPEAN PATENT APPLICATION

(11) **EP 4 737 891 A1**
(43) Date of publication of application: **06.05.2026**
(21) Application number: 25210655.4
(22) Date of filing: 23.10.2025
(51) Int. Cl.: G01N 24/08, G01N 21/64, G01R 33/46, G01R 33/465, A61K 9/51

(54) **METHOD FOR PREDICTING TRANSFECTION EFFICIENCY OF FLUORINATED LIPID NANOPARTICLES USING NUCLEAR MAGNETIC RESONANCE**

(30) Priority: 03.11.2024 CN 202411553684
(71) Applicant: Innovation Academy for Precision Measurement Science and Technology, CAS, Wuhan, Hubei 430071 (CN)
(72) Inventor: ZHOU, Xin, Wuhan City (CN); XIE, Kairu, Wuhan City (CN); CHEN, Daiqin, Wuhan City (CN); JIANG, Zhongxing, Wuhan City (CN); ZHU, Lijun, Wuhan City (CN); CHEN, Shizhen, Wuhan City (CN)
(74) Representative: Murgitroyd & Company

(57) **Abstract**

The present invention relates to the field of analytical detection, and provides a method for predicting the transfection efficiency of fluorinated lipid nanoparticles (FLNPs) by utilizing nuclear magnetic resonance. The method includes the following steps repeated for multiple FLNPs containing different fluorine contents:

(1) performing 19F magnetic resonance spectroscopy (19F MRS) on the FLNPs to obtain a fluorine spectrum of a control group; (2) transfecting the FLNPs into cells or living mice and performing 19F MRS to obtain a fluorine spectrum of an experimental group; (3) observing the transfection of the FLNPs using a confocal fluorescence microscope to quantify fluorescence intensity; (4) generating a calibration curve correlating post-transfection fluorescence intensity with the difference in the target peak area of the fluorinated lipid between the experimental and control groups; and (5) predicting FLNPs transfection using the calibration curve. This approach demonstrates excellent specificity, reproducibility, stability, and linearity, providing a robust technical foundation for large-scale screening of FLNPs-based nanomedicines.

## Description

### TECHNICAL FIELD

The present invention relates to the technical field of analytical detection, and in particular to a method for predicting transfection efficiency of fluorinated lipid nanoparticles by utilizing multi-nuclear magnetic resonance.

### BACKGROUND

Nucleic acid therapies have great potential in the treatment and prevention of diseases. Messenger ribonucleic acid-based technologies have achieved considerable clinical effects in vaccines, protein supplementation therapies, and gene editing therapies (Han X, Xu J, Xu Y, et al. Nat Commun. 2024; 15(1): 1762.). However, nucleic acids are negatively charged and unstable macromolecules that require carriers for efficient intracellular delivery.

Lipid nanoparticles (LNPs) are the most advanced non-viral nucleic acid delivery platform in clinic currently. Studies have shown that only a small amount of nucleic acids carried by the LNPs can escape lysosomes and enter the cytoplasm (Gilleron J, Querbes W, Zeigerer A, et al. Nat Biotechnol. 2013; 31(7): 638-646.). In order to further improve the nucleic acid delivery performance of the LNPs, fluorinated lipids are used for improving the efficiency of nucleic acid delivery in cells, which is mainly attributable to the fact that the fluorinated lipids added into fluorinated LNPs can significantly promote the cellular uptake and lysosomal escape of the LNPs (Zhang H, Meng C, Yi X, et al. ACS Nano. 2024; 18(11): 7825-7836.). After the fluorinated LNPs successfully escape the lysosomes, they will release the carried nucleic acids and fluorinated lipids, which facilitates the subsequent nucleic acid transfection and protein expression. Therefore, the release of the fluorinated lipids is correlated with the subsequent protein expression.

Currently, the most intuitive method for detecting LNP transfection efficiency at an *in vitro* level is to observe the expression of fluorescent proteins using a confocal microscope. However, this method cannot avoid the differences in LNP delivery *in vitro* and *in vivo.* The most commonly used method for detecting LNP transfection efficiency at an *in vivo* level is to inject LNPs into mice for a certain period of time, and then acquire tissues from the injection sites of the mice for immunofluorescence staining to analyze protein expression. However, this method requires the sacrifice of a large number of mice, so usually only a small number of LNPs can be screened *in vivo.*

### SUMMARY

Based on the aforementioned situation, an objective of the present invention is to provide a method for predicting transfection efficiency of fluorinated lipid nanoparticles (FLNPs) by detecting fluorine spectra before and after transfection into cells or living mice using magnetic resonance spectroscopy (MRS). This method addresses limitations of conventional techniques, which often involve inaccurate measurements and complex procedures. The proposed approach enables simple, rapid, and accurate prediction of FLNPs transfection efficiency, facilitating large-scale screening of FLNPs in non-human primate models.

To achieve the objective of the present invention, the following technical solution is proposed:

The present invention provides a method for predicting transfection efficiency of FLNPs using multi-nuclear magnetic resonance analysis. The method includes the following steps:
(1) lysing untransfected cells to obtain a cell lysate, mixing the cell lysate with the FLNPs, and performing ¹⁹F MRS to obtain the fluorine spectrum of a control group;
(2) transfecting the FLNPs into cells (preferably 293T cells), lysing the transfected cells to obtain a lysate, and performing ¹⁹F MRS to obtain the fluorine spectrum of an experimental group;
(3) identifying the characteristic fluorine peak of the fluorinated lipid from both the control and experimental ¹⁹F MRS obtained in steps (1) and (2), and calculating the difference in the target peak area between the two groups;
(4) observing the transfection of the FLNPs following step (2) using a confocal fluorescence microscope, and quantifying the fluorescence intensity;
(5) repeating steps (1)-(4) using FLNPs containing different fluorine contents;
(6) plotting a calibration curve with the difference in the target peak area of the fluorinated lipid between the experimental and control groups on the x-axis, and the corresponding post-transfection fluorescence intensity on the y-axis, to establish a linear correlation between the two parameters; and
(7) for any FLNPs to be evaluated, performing steps (1)-(3) and determining their transfection efficiency based on the calibration curve obtained in step (6).

In the aforementioned method of the present invention, the cells can also be replaced with living experimental mice, and in this case, the aforementioned steps (1) and (2) are respectively:
(1-1) performing ¹⁹F MRS on the FLNPs to obtain the fluorine spectrum of the control group; and
(2-1) injecting the FLNPs into mice, and after a defined incubation period, performing ¹⁹F MRS at the injection site to obtain the fluorine spectrum of the experimental group.

The FLNPs are obtained by a conventional method, and their composition at least includes fluorinated lipids and plasmids, and a structure of the included fluorinated lipids is as follows: 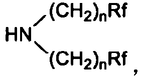 where, Rf is selected from any one of perfluoro-tert-butoxy, trifluoroethoxy, difluoroethoxy, pentafluorothiobenzyloxy, p-fluorobenzyloxy, fluorobenzyloxy such as difluorobenzyloxy, bistrifluoromethylbenzyloxy, trifluoromethoxybenzyloxy, trifluoromethylthiobenzyloxy, and linear polyfluoroalkyl having a structure of -O(CH₂)₂(CF₂)nCF₃, where n is a natural number of 3 to 20, and preferably bis(12-(perfluoro-*tert*-butoxyl)dodecyl)amine (n is 12, Rf is perfluoro-*tert*-butoxy).

Preferably, the FLNPs are prepared by the following method: mixing an ethanol solution containing bis(12-(perfluoro-*tert*-butoxyl)dodecyl)amine, heptadecan-9-yl 8-((2-hydroxyethyl)(6-oxo-6-(undecyloxy)hexyl)amino)octanoate) , 1,2-distearoyl-sn-glycero-3-phosphocholine , cholesterol and 1,2-dimyristoyl-rac-glycerol-3-methoxy polyethylene glycol-2000 with a pEGFP plasmid solution containing an EGFP-encoding plasmid at a certain volume ratio (preferably 1:3), and a molar ratio of bis(12-(perfluoro-*tert-*butoxyl)dodecyl)amine, heptadecan-9-yl 8-((2-hydroxyethyl)(6-oxo-6-(undecyloxy)hexyl)amino)octanoate), 1,2-Distearoyl-sn-glycero-3-phosphocholine, cholesterol and 1,2-dimyristoyl-rac-glycerol-3-methoxy polyethylene glycol-2000 is (12.5 to 37.5):(12.5 to 37.5):10:38.5:1.5; and a mass ratio of the plasmid to the cholesterol is 150 µg:0.6 mg.

In order to conduct a more accurate quantitative analysis of the fluorine contents in the step (1) and the step (2), before the detection in the step (1) and the step (2), an external standard dissolved in a deuterated reagent is added to the corresponding cell lysate, where the deuterated reagent comprises any one of deuterated water, deuterated methanol, deuterated acetonitrile, and deuterated dimethyl sulfoxide, and preferably deuterated water; and
the external standard includes any one of trifluoroacetic acid, 2-chloro-3-fluoropyridine, sodium trifluoromethanesulfonate and 4-fluorobenzoic acid, and preferably sodium trifluoromethanesulfonate, and the chemical shift of its target peak is 75-85 ppm, and preferably 78-80 ppm. As a preferred technical solution of the present invention, using sodium trifluoromethanesulfonate as an external standard can ensure the stability and accuracy of a characteristic peak target integration result. Also, sodium trifluoromethanesulfonate and bis(12-(perfluoro-*tert*-butoxyl)dodecyl)amine each produce only one corresponding fluorine signal, which is close and completely separated, without any interference phenomenon, further improving the accuracy of an integrated area result of the characteristic target peak.

Preferably, a concentration of the external standard dissolved in the deuterated reagent is 0.5 to 5 mM, and preferably 1 mM; and in the step (1) and the step (2), a volume ratio of the corresponding cell lysate to the external standard is 1:(0.02 to 0.4), and preferably 10:1.

Preferably, a frequency of the nuclear magnetic resonance spectrometer used in the nuclear magnetic resonance fluorine spectrum test is 400 to 600 MHz.

Preferably, the nuclear magnetic resonance fluorine spectrum detection adopts a zgig pulse sequence or a rapid acquisition with relaxation enhancement sequence, and a test temperature is 297 ± 2 K; and/or
a pulse width during the nuclear magnetic resonance fluorine spectrum detection is 11 to 12 µs, and preferably 11.1 to 11.5 µs; and/or
a relaxation time during the nuclear magnetic resonance fluorine spectrum detection is 10 to 20 s, and preferably 14 to 16 s; and/or
a spectral width of the nuclear magnetic resonance fluorine spectrum detection is 7,000 to 8,000 Hz, and preferably 7,300 to 7,500 Hz; and/or
a radio frequency center frequency during the nuclear magnetic resonance fluorine spectrum detection is -45,000 to -35,000 Hz, and preferably -41,000 to -40,000 Hz; and/or
a number of acquisition points during the nuclear magnetic resonance fluorine spectrum detection is 125,000 to 135,000, and preferably 130,000 to 132,000; and/or
a sampling time during the nuclear magnetic resonance fluorine spectrum detection is 7 to 10 s, and preferably 8 to 9 s; and/or
the number of samplings during the nuclear magnetic resonance fluorine spectrum detection is 20 to 40, and preferably 30 to 35; and/or
the number of empty scans during the nuclear magnetic resonance fluorine spectrum detection is 3 to 6, and preferably 3 to 5; and/or
a gain during the nuclear magnetic resonance fluorine spectrum detection is 60 to 150, and preferably 120 to 135.

In the method for determining the content of fluorinated lipids described in the present invention, considering that a response signal range of the fluorine spectrum is relatively wide, in order to reduce the error of phase adjustment and ensure the accuracy of integration, the radio frequency center frequency is set at a middle position between an external standard peak and a sample peak to ensure the accuracy of the target peak integration.

Compared with the prior art, the advantages and beneficial effects of the present invention are:

The present invention employs ¹⁹F MRS to analyze FLNPs and their fluorine spectra after transfection into the cells or living mice, enabling the prediction of FLNPs transfection efficiency. Specifically, the method quantifies fluorinated lipid content using ¹⁹F MRS. Through optimization of experimental parameters, the detection process achieves simplicity, high reproducibility, stability, and accuracy, making it suitable for routine application. The use of sodium trifluoromethanesulfonate as an external standard ensures reliable integration of characteristic fluorine peaks and accurate quantification of fluorinated lipid content. Overall, this method demonstrates excellent specificity, precision, repeatability, and linearity, meeting the requirements for predicting FLNPs transfection efficiency both *in vitro* and *in vivo,* and providing a robust foundation for large-scale screening of FLNPs.

### Brief Description of the Drawings

FIG. 1 shows ¹⁹F MRS before and after cells are transfected with FLNPs in Example 2;
FIG. 2 is a representative confocal image after the cells are transfected with the FLNPs in Example 3;
FIG. 3 is a statistical graph of fluorescence intensity after the cells are transfected with the FLNPs;
FIG. 4 shows a linear relationship between the peak area difference of the target fluorine spectra before and after the cells are transfected with the FLNPs and the fluorescence intensity;
FIG. 5 shows a ¹⁹F MRS before and after FLNPs are injected into the gastrocnemius muscle of the mice; and
FIG. 6 is a representative image of immunofluorescence-stained sections of the gastrocnemius muscle of the mice 48 h after injection of the FLNPs.

### DETAILED DESCRIPTION OF THE INVENTION

The technical solution of the present invention is described in detail hereafter with reference to specific embodiments and drawings.

The main reagents and materials used in the following examples are described as follows: 293T cells are purchased from the Shanghai Cell Bank of the Chinese Academy of Sciences. A RIPA lysis buffer is purchased from Boster (catalog number AR0102). Sodium trifluoromethanesulfonate is purchased from J&K Scientific, Beijing. 5 mm nuclear magnetic resonance tubes are purchased from Wilmad, USA. 500 MHz and 400 MHz AVANCE NEO-type nuclear magnetic resonance spectrometers are purchased from Bruker, Switzerland. 1 mm capillary tubes are purchased from the Instrument Factory of West China University of Medical Sciences. heptadecan-9-yl 8-((2-hydroxyethyl)(6-oxo-6-(undecyloxy)hexyl)amino)octanoate) is purchased from Sinopeg Biotech Co., Ltd., Xiamen, 1,2-Distearoyl-sn-glycero-3-phosphocholine and 1,2-dimyristoyl-rac-glycerol-3-methoxy polyethylene glycol-2000 are from Ruixi Biotech, and cholesterol is purchased from Sigma.

A solution containing pEGFP plasmids encoding EGFP proteins is prepared: a high-purity plasmid Maxi extraction kit is utilized to extract the EGFP-expressing plasmids from *Escherichia coli,* and then the plasmids were diluted to 1 mg/mL with a 50 mM citrate buffer (pH = 3) for subsequent experiments. The high-purity plasmid Maxi extraction kit is purchased from Tiangen Biotech (Beijing) Co., Ltd. DP116.

Example 1 Preparation of FLNPs. The specific steps were as follows:
(1) An ethanol solution containing bis(12-(perfluoro-*tert*-butoxyl)dodecyl)amine (abbreviated as F12C), heptadecan-9-yl 8-((2-hydroxyethyl)(6-oxo-6-(undecyloxy)hexyl)amino)octanoate) (abbreviated as SM102), 1,2-Distearoyl-sn-glycero-3-phosphocholine (abbreviated as DSPC), cholesterol (abbreviated as Chol) and 1,2-dimyristoyl-rac-glycerol-3-methoxy polyethylene glycol-2000 (abbreviated as DMG-PEG 2000) was mixed with 1.2158 mL of the pEGFP plasmid solution prepared above (containing 150 µg of plasmids) at a volume ratio of 1:3 to prepare a total of four LNPs, i.e. LNP-SM102, LNP-25%F12C, LNP-50%F12C, and LNP-75%F12C, respectively. Then they were diluted with 1 × PBS (0.0067 M, the same below) for subsequent cell experiments. The molar ratio of F12C, SM102, DSPC, Cholesterol and DMG PEG 2000 was shown in Table 1 (for example, the formula of the LNP-SM102 group was: 1.5 mg of SM102, 0.33 mg of DSPC, 0.6 mg of Chol and 0.16 mg of DMG-PEG2000 dissolved in 405.3 µL of absolute ethanol, and in other groups, the dosages of Chol and DMG-PEG2000 were kept unchanged, while the dosages of other substances were adjusted):

**Table 1**

| LNP Name | Formula | Molar Ratio |
|---|---|---|
| LNP-SM102 | SM102/DSPC/ Chol/DMG-PEG2000 | 50: 10: 38.5: 1.5 |
| LNP-25%F12C | F12C/SM102/DSPC/ Chol/DMG-PEG2000 | 12.5: 37.5: 10: 38.5: 1.5 |
| LNP-50%F12C | F12C/SM102/DSPC/ Chol/DMG-PEG2000 | 25: 25: 10: 38.5: 1.5 |
| LNP-75%F12C | F12C/SM102/DSPC/ Chol/DMG-PEG2000 | 37.5: 12.5: 10: 38.5: 1.5 |

(2) Characterization data of the four LNPs were shown in Table 2:

**Table 2**

| LNP Name | Hydrated particle size (nm) | PDI |
|---|---|---|
| LNP-SM102 | 106.5 ± 12.7 | 0.132 |
| LNP-25%F12C | 96.7 ± 12.7 | 0.176 |
| LNP-50%F12C | 116.8 ± 7.9 | 0.188 |
| LNP-75%F12C | 91.9 ± 11.0 | 0.212 |

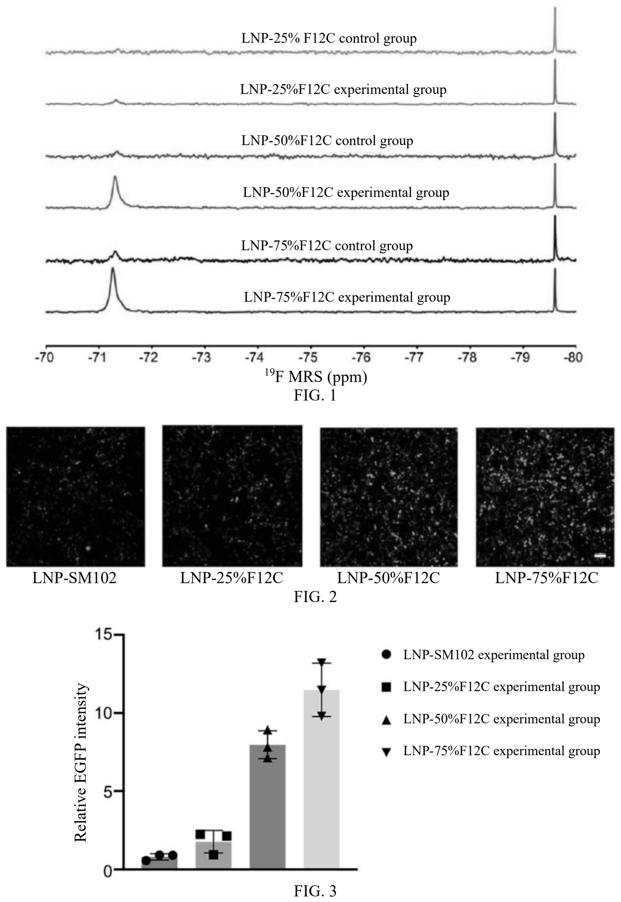

Example 2 Calculation of the difference in target peak area of the ¹⁹F MRS before and after the cells were transfected with the FLNPs. The specific steps were follows:
(1) 293T cells were transfected respectively with the LNP-25%F12C, the LNP-50%F12C and the LNP-75%F12C prepared in Example 1 (with 98 µg of pEGFP in LNP of each group), and after 48 h, about 5E + 7 cells were collected in each group. The cells were lysed with 1 mL of a RIPA lysis buffer, and centrifuged at 12,000 g. Then the supernatant was taken and loaded into a new EP tube for later use, so as to obtain the cell lysate of the LNP-25%F12C experimental group, the cell lysate of the LNP-50%F12C experimental group and the cell lysate of the LNP-75%F12C experimental group.
(2) To reduce the influence of variables, 5E + 7 untreated 293T cells were lysed with 900 µL of a RIPA lysis buffer, and centrifuged at 12,000 g, and then the supernatant was taken and loaded into a new EP tube for later use, so as to obtain the cell lysate of the control group.
(3) Preparation of capillary external standard: 0.172 mg of sodium trifluoromethanesulfonate was taken and dissolved in 1 mL of deuterated water to prepare a sodium trifluoromethanesulfonate solution at a concentration of 1 mM. 50 µL of the solution was taken and loaded into a capillary tube with a diameter of 1 mm using a microsyringe. Both ends of the capillary tube were sealed to obtain the sodium trifluoromethanesulfonate external standard.
(4) each 500 µL of the cell lysate of the experimental group obtained in the step (1) was taken and mixed with the sodium trifluoromethanesulfonate external standard obtained in the step (3), and the mixture was transferred into a nuclear magnetic resonance tube with a diameter of 5 mm to obtain a detection solution of the LNP-25%F12C experimental group, a detection solution of the LNP-50%F12C experimental group and a detection solution of the LNP-75%F12C. Nuclear magnetic resonance detection was performed using a 500 MHz VANCE NEO-type nuclear magnetic resonance spectrometer to obtain the nuclear magnetic resonance fluorine spectrograms of the LNP-25%F12C experimental group, the LNP-50%F12C experimental group, and the LNP-75%F12C experimental group (FIG. 1), respectively;
(5) The cell lysate of the control group obtained in the step (2) (about 900 µL) was mixed with the LNP-25%F12C, LNP-50%F12C and LNP-75%F12C obtained and diluted in the step (1) (with 98 µg of pEGFP in each LNP, and the volume was about 100 µL), respectively. Each 500 µL of the mixed solution was taken and mixed with the sodium trifluoromethanesulfonate external standard obtained in the step (3), and the mixture was transferred into a nuclear magnetic resonance tube with a diameter of 5 mm to obtain a detection solution of the LNP-25%F12C control group, a detection solution of the LNP-50%F12C control group and a detection solution of the LNP-75%F12C control group. Nuclear magnetic resonance detection was performed using a 500 MHz VANCE NEO-type nuclear magnetic resonance spectrometer to obtain the ¹⁹F MRS of the LNP-25%F12C control group, the LNP-50%F12C control group, and the LNP-75%F12C control group (FIG. 1), respectively.

The sampling parameters for the ¹⁹F MRS in this example were as follows: a zgig pulse sequence was adopted, the test temperature was 297 K, the pulse width was 11.1 µs, the relaxation time was 15 s, the spectral width was 7,462.8 Hz, the radio frequency center frequency was -40,664.92 Hz, the number of acquisition points was 131,000, the sampling time was 8.78 s, the number of samplings was 32, the number of empty scans was 4, and the gain was 128.

The ¹⁹F MRS obtained in the step (4) and the step (5) were shown in FIG. 1, where the characteristic peak with a chemical shift of -79.6 ppm was for the external standard sodium trifluoromethanesulfonate, and the characteristic peak with a chemical shift of 71.3 ppm was for F12C (fluorinated lipids).

(6) The data of ¹⁹F MRS was processed via MestReNova v14.2.3 software. After baseline calibration and phase adjustment, the target peaks of F12C and the external standard were integrated. The target peak area of the external standard was set to 1. The target peak area of the fluorinated lipids in the experimental group was represented as *Sₐ,* and the target peak area of the fluorinated lipids in the control group was represented as *S_{b}.* The difference between the target peak area of the fluorinated lipids in the experimental group and the target peak area of the fluorinated lipids in the control group was represented as *S_{Δ}.* The calculation formula was: *S_{Δ} = Sₐ - S_{b}.* The difference between the target peak area of the fluorinated lipids in the experimental group and the target peak area of the fluorinated lipids in the control group was shown in Table 3:

**Table 3**

| LNP Name | *S_{Δ}* |
|---|---|
| LNP-25%F12C | 0.35 |
| LNP-50%F12C | 4.64 |
| LNP-75%F12C | 6.71 |

Example 3 Cell transfection efficiency of different FLNPs as observed by a confocal fluorescence microscope. The specific steps were as follows:
(1) 293T cells were transfected using the LNPs prepared in Example 1. Specifically, an appropriate number of 293T cells were seeded in a glass-bottom confocal dish with a diameter of 2 cm and allowed for cell confluence (equally divided into a LNP-SM102 group, a LNP-25%F12C group, a LNP-50%F12C group, and a LNP-75%F12C group). 12 h later, the corresponding diluted LNPs were added to the confocal dish of each group. The LNP volume was 19.6 µL, and the pEGFP content was 0.39 µg. The cells were incubated in an environment at 37°C and 5% CO₂ for 48 h, then the culture medium was removed, the cells were fixed with paraformaldehyde, and the cell nuclei were stained with a staining agent DAPI. The fluorescence appearance was photographed by using a fluorescence confocal microscope (FIG. 2, scale = 100 µm).
(2) The photographed fluorescence images were analyzed using Image J software for the fluorescence expression intensity (FIG. 3).

Example 4 The linear relationship between the difference in the peak area of the target fluorine spectra before and after the cells were transfected with the fluorinated LNPs and the fluorescence intensity. The specific steps were follows:

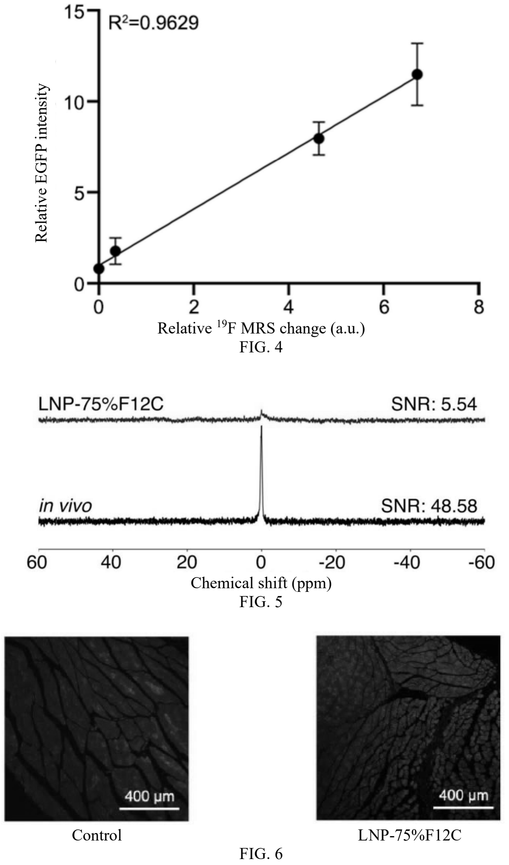

The difference in target peak area of nuclear magnetic resonance fluorine spectra before and after the cells were transfected with the fluorinated LNPs obtained in Example 2 was used as the X axis, and the fluorescence expression intensity of the cells transfected with the fluorinated LNPs obtained in Example 3 was used as the Y axis (LNP-SM102 contained no fluorinated lipid, that was, the X axis was counted as 0 and the fluorescence intensity was used as the Y axis). The fitting coefficient (R²) obtained by linear regression analysis was used (FIG. 4).

Analysis of the results showed that higher fluorescence intensity after transfection with the fluorinated LNPs corresponded to a greater difference in the target fluorine peak area before and after cell transfection. Linear regression analysis yielded a model fitting coefficient of 0.9629, indicating a strong correlation. These findings demonstrate that the difference in peak area of the target fluorine spectra before and after the cells were transfected with the FLNPs could be used for predicting the transfection efficiency of the FLNPs.

Example 5: Detection of ¹⁹F MRS before and after the FLNPs were injected into mice, and measurement of the corresponding protein expression, was conducted using the following steps:
(1) ¹⁹F MRS was performed on LNP-75%FC12 prepared in Example 1 to obtain the fluorine spectrum prior to injection of the FLNPs (FIG. 5, LNP-75%F12C);
(2) The LNP-75%F12C obtained in Example 1 was injected into the gastrocnemius muscle of Balb/c nude mice, with 50 µL injected into the gastrocnemius muscle unilaterally. The mice injected intramuscularly with 50 µL of PBS served as a control group. After 48 h, the mice were anesthetized by intraperitoneal injection of 150 µL of a 1% pentobarbital sodium solution, and the ¹⁹F MRS of the mouse legs was acquired using a 400 MHz AVANCE NEO-type nuclear magnetic resonance spectrometer to obtain the post-injection nuclear magnetic resonance fluorine spectrum (FIG. 5, *in vivo*);

The sampling sequence was a rapid acquisition with relaxation enhancement (RARE) sequence with the following sampling parameters: a repetition time of 500 ms; an average number of 18,000; and a repetition number of 1.

(3) The mice were dissected and their gastrocnemius muscle tissues were fixed in a 4% paraformaldehyde solution for 24 h. Then the gastrocnemius muscle tissues were then embedded in paraffin and sliced, and subjected to immunofluorescence staining. Afterwards, the EGFP protein expressed in the gastrocnemius muscle tissues was observed using a confocal fluorescence microscope (FIG. 6, where the control was the control group).

The difference in the target fluorine peak area before and after injection of the FLNPs into mice was substantial, with signal-to-noise ratios (SNR) of 48.58 and 5.54, respectively, indicating successful release of nucleic acids and fluorinated lipids, consistent with the *in vitro* results. Furthermore, immunofluorescence staining of mouse gastrocnemius muscle sections revealed robust EGFP protein expression, demonstrating the effectiveness of this vector for nucleic acid delivery and protein expression. Collectively, these findings confirm that ¹⁹F MRS can be used to predict the *in vivo* transfection efficiency of FLNPs.

## Claims

1. A method for predicting transfection efficiency of fluorinated lipid nanoparticles (FLNPs) by utilizing multi-nuclear magnetic resonance, comprising the following steps:
(1) lysing untransfected cells to obtain a cell lysate, mixing the cell lysate with the FLNPs, and performing ¹⁹F magnetic resonance spectroscopy (MRS) to obtain the fluorine spectrum of a control group;
(2) transfecting the FLNPs into cells, lysing the transfected cells to obtain a lysate, and performing ¹⁹F MRS to obtain the fluorine spectrum of an experimental group;
(3) identifying the characteristic fluorine peak of the fluorinated lipid from both the control and experimental ¹⁹F MRS obtained in steps (1) and (2), and calculating the difference in the target peak area between the two groups;
(4) observing the transfection of the FLNPs following step (2) using a confocal fluorescence microscope, and quantifying the fluorescence intensity;
(5) repeating steps (1)-(4) using FLNPs containing different fluorine contents;
(6) plotting a calibration curve with the difference in the target peak area of the fluorinated lipid between the experimental and control groups on the x-axis, and the corresponding post-transfection fluorescence intensity on the y-axis, to establish a linear correlation between the two parameters; and
(7) for any FLNPs to be evaluated, performing steps (1)-(3) and determining their transfection efficiency based on the calibration curve obtained in step (6);
wherein the FLNPs are obtained by a conventional method, and their composition at least comprises fluorinated lipids and plasmids, and a structure of the comprised fluorinated lipids is as follows: 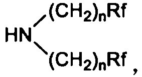
wherein, Rf is selected from any one of perfluoro-*tert*-butoxy, trifluoroethoxy, difluoroethoxy, pentafluorothiobenzyloxy, p-fluorobenzyloxy, difluorobenzyloxy, bistrifluoromethylbenzyloxy, trifluoromethoxybenzyloxy, trifluoromethylthiobenzyloxy, and linear polyfluoroalkyl having a structure of -O(CH₂)₂(CF₂)nCF₃, wherein n is a natural number of 3 to 20.

2. The method according to claim 1, wherein the cells in the method are replaced with living experimental mice, and in this case, the aforementioned steps (1) and (2) are respectively:
(1-1) performing ¹⁹F MRS on the FLNPs to obtain the fluorine spectrum of the control group; and
(2-1) injecting the FLNPs into mice, and after a defined incubation period, performing ¹⁹F MRS at the injection site to obtain the fluorine spectrum of the experimental group.

3. The method according to claim 1, wherein before the detection in the step (1) and the step (2), an external standard dissolved in a deuterated reagent is added to the corresponding cell lysate, wherein the deuterated reagent comprises any one of deuterated water, deuterated methanol, deuterated acetonitrile, and deuterated dimethyl sulfoxide; and
the external standard is any one of trifluoroacetic acid, 2-chloro-3-fluoropyridine, sodium trifluoromethanesulfonate, and 4-fluorobenzoic acid.

4. The method according to claim 3, wherein a concentration of the external standard dissolved in the deuterated reagent is 0.5 to 5 mM; and in the step (1) and the step (2), a volume ratio of the corresponding cell lysate to the external standard is 1:(0.02 to 0.4).

5. The method according to any one of claims 1-4, wherein a frequency of the nuclear magnetic resonance spectrometer used in the nuclear magnetic resonance fluorine spectrum test is 400 to 600 MHz.

6. The method according to claim 5, wherein the nuclear magnetic resonance fluorine spectrum detection adopts a zgig pulse sequence or a rapid acquisition with relaxation enhancement sequence, and a test temperature is 297 ± 2 K; and/or
a pulse width during the nuclear magnetic resonance fluorine spectrum detection is 11 to 12 µs; and/or
a relaxation time during the nuclear magnetic resonance fluorine spectrum detection is 10 to 20 s; and/or
a spectral width of the nuclear magnetic resonance fluorine spectrum detection is 7,000 to 8,000 Hz; and/or
a radio frequency center frequency during the nuclear magnetic resonance fluorine spectrum detection is -45,000 to -35,000 Hz; and/or
a number of acquisition points during the nuclear magnetic resonance fluorine spectrum detection is 125,000 to 135,000; and/or
a sampling time during the nuclear magnetic resonance fluorine spectrum detection is 7 to 10 s; and/or
the number of samplings during the nuclear magnetic resonance fluorine spectrum detection is 20 to 40; and/or
the number of empty scans during the nuclear magnetic resonance fluorine spectrum detection is 3 to 6; and/or
a gain during the nuclear magnetic resonance fluorine spectrum detection is 60 to 150.
